# EUROPEAN PATENT SPECIFICATION

(11) **EP 3 030 228 B1**
(45) Date of publication and mention of the grant of the patent: **02.09.2020**
(21) Application number: 14834998.8
(22) Date of filing: 31.07.2014
(51) Int. Cl.: A61K 9/34, A61K 33/10, A61K 33/00, A61K 31/047, A61K 36/185, A61P 1/04

(54) **REDUCING REFLUX WHILE SLEEPING BY STIMULATING SALIVA WITH ADHERING TROCHES**
REFLUXREDUZIERUNG WÄHREND DES SCHLAFES DURCH SPEICHELSTIMULIERUNG MIT ANHAFTENDEN TABLETTEN
RÉDUCTION DE REFLUX PENDANT LE SOMMEIL PAR STIMULATION DE SALIVE À L'AIDE DE TROCHISQUE ADHÉRANT

(30) Priority: 08.08.2013 US 201361863836 P
(43) Date of publication of application: 15.06.2016
(73) Proprietor: Quest Products, LLC, Pleasant Prairie, WI 53158 (US)
(72) Inventor: HALEY, Jeffrey, Bellevue, Washington 98005 (US)
(74) Representative: Gerli, Paolo
(86) International application number: PCT/IB2014/063568
(87) International publication number: WO 2015/019246

(56) References cited:
- EP-A1- 2 462 926
- EP-A2- 0 413 427
- WO-A1-2007/139661
- US-A1- 2010 285 098
- US-A1- 2011 177 174
- US-B1- 6 428 827
- HERRERA J L ET AL: "SALIVA: ITS ROLE IN HEALTH AND DISEASE", JOURNAL OF CLINICAL GASTROENTEROLOGY, RAVEN PRESS LTD., NEW YORK, NY, US, vol. 10, no. 5, 1 October 1988 (1988-10-01), pages 569-578, XP008015992, ISSN: 0192-0790

## Description

### BACKGROUND

Gastro-esophageal reflux disease (GERD), also called acid reflux, is caused by stomach acids rising into the esophagus. If untreated, it can cause an ulcer of the esophagus, a type of peptic ulcer, can cause cancer, and can erode teeth. GERD is typically worst while sleeping when the body is reclined so that gravity does not hold stomach acid down and swallowing is infrequent because saliva flow is lowest while sleeping. During the day, one can reduce acid reflux by stimulating saliva and therefore frequent swallowing through the use of chewing gum or slowly dissolving lozenges.

### SUMMARY OF THE INVENTION

The present invention provides an adhering, bilayer troche for use in a method of reducing reflux from a stomach toward a mouth while sleeping in a patient in need thereof. The adhering, bilayer troche provided to the patient comprises: a first layer comprising an adhesive powder, and a second layer comprising a substrate that slowly dissolves or erodes in saliva and an ingredient to be released, wherein the ingredient stimulates saliva production in the mouth. The patient is instructed to adhere one or more of said troches in the mouth before going to sleep, thereby causing frequent swallowing of stimulated saliva while sleeping resulting in reduced reflux from the stomach.

In another aspect, the invention provides a slowly dissolving, adhering, bilayer troche for use in a method of reducing reflux from a stomach toward a patient's mouth while sleeping, wherein said method comprises: adhering in the mouth before going to sleep said troche that dissolves while sleeping and releases an ingredient that stimulates saliva production while sleeping, thereby causing frequent swallowing of stimulated saliva resulting in reduced reflux from the stomach, wherein the troche comprises: a first layer comprising an adhesive powder, and a second layer comprising a substrate that slowly dissolves or erodes in saliva and an ingredient to be released, wherein the ingredient stimulates saliva production in the mouth.

In another aspect, a kit is described. The kit comprises, a bilayer, adhering troche, wherein the troche comprises a first layer comprising an adhesive powder, and a second layer comprising a substrate that slowly dissolves or erodes in saliva and an ingredient to be released, wherein the ingredient stimulates saliva production in the mouth thereby causing frequent swallowing of stimulated saliva resulting in reduced reflux from the stomach; and instructions to adhere one or more of the troches in the mouth before sleeping.

In embodiments, the troche may be from about 5 mm to about 18 mm in each of two dimensions. In embodiments, the ingredient that stimulates saliva production comprises flavour molecules. Flavour molecules may comprise a sweet polyol, including xylitol, sorbitol, maltitol, erythritol and mannitol, or a synthetic sweetener, including sucralose, neotame, aspartame, acesulfame potassium and saccharin, or stevia.

In embodiments, the person is instructed to adhere one or more troches to an outside of a molar or gum adjoining the outside of the molar.

### BRIEF DESCRIPTION OF THE DRAWING

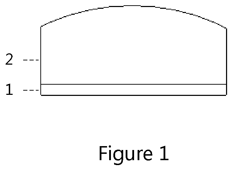

Figure 1 shows a side view of a bi-layer adhering troche made with a tablet press.

### DETAILED DESCRIPTION

A troche with a slowly dissolving substrate and an ingredient that stimulates saliva may be made by mixing dry granular powders. The substrate comprises at least one powder that will slowly dissolve or erode in saliva, such as a carbohydrate like inulin, r polydextrose or a polyol.

The ingredient may be flavor molecules that comprise any combination of sweet, sour, salty, bitter or savory. All of these flavors will stimulate saliva production. Typically, flavors are sweet, savory and salty.

If a sweet flavor is used, it is desirable to not use a cariogenic carbohydrate like sugar because these carbohydrates promote tooth decay. Some suitable non-cariogenic carbohydrates include polyols, e.g., xylitol, sorbitol, maltitol, erythritol, and mannitol. Stevia and the synthetic sweeteners such as sucralose, neotame, aspartame, acesulfame potassium, and saccharin are also suitable.

The troches may be formed by pressing powders into a tablet as shown in Figure 1 by using a bi-layer tablet press. Troches will generally be at least 5 mm diameter if round, and at least 5 mm in each of two dimensions if not round. Generally, troches will be from 5-18 mm diameter or 5-18 mm in each of two dimensions. Grain sizes of 50 to 350 microns are typical. The grains may be granulated with a coating of binder gum on the outside, such as Danisco Xylitab® 200 which is granulated with up to 2% carboxymethylcellulose (CMC - cellulose gum) as a compression binder. Alternatively, grains of material not coated with a gum, such as Danisco Xylitab® 300, may be mixed with a binder gum powder such as CMC and then pressed. Some of the polyols, such as sorbitol and maltitol, compress well and may be used without a compression binder.

The adhesive molecules may comprise one or more of acacia gum, gelatin, alginate, starch, pectin, polyvinylpyrolidone, carboxymethylcellulose, hydroxymethylcellulose, polyvinyl acid, polyacrylic acid, and carbopol. Acacia gum adheres very well to teeth and gingiva, and it does not dissolve too fast or leave an unattractive mouth feel. On the surface designed to be adherent, between 80% and 100% acacia gum is preferred for good adhesion. Acacia gum may be mixed with an alkalizer to obtain and keep it pH neutral. A suitable ratio for pH neutrality is 1 unit calcium carbonate (CaCO3) to 30 units acacia gum. Alternatively, the adhesive molecules may comprise one or more of gelatin, alginate, starch, pectin, polyvinylpyrolidone, carboxymethylcellulose, hydroxymethylcellulose, polyvinyl acid, polyacrylic acid, and carbopol. Percentages of these molecules that exhibit adherent function are well known.

The adherent layer can be quite thin. In tests on a troche of about 12 mm in diameter by 4 to 5 mm thick, a suitable thickness of a layer of about 97% acacia gum was about 0.5 - about 0.9 mm.

An adhering troche held in a human mouth erodes, releasing flavor molecule over time, allowing delivery of saliva-stimulating ingredients without the effect on the appearance of chewing or having a candy in one's mouth. Moreover, an adhering troche can be used safely while sleeping. While awake, a 0.7 g troche of xylitol with 4% CMC and about 4.5 mm thick dissolves in a human mouth with normal saliva flow in 30-70 minutes, although the time depends at least in part on saliva flow, saliva chemistry, and mouth movement. While asleep, a troche having the same composition lasts generally at least 2-8 hours.

An exemplary method for making bi-layer troches using a typical press, comprises placing the ingredient-releasing powder in the die, sitting on the lower punch, tamping the powder with the upper punch, which leaves the surface having the shape of the upper punch face, adding powder of the adhesive layer, and pressing with an upper punch. The shape of the upper punch that presses the ingredient-releasing powder and that presses the adhesive powder may be the same or different. Thus, a troche may have an adhesive side and an ingredient side with different shapes. For example, the ingredient-releasing powder may be tamped with a flat or essentially flat or rounded punch and the adhesive powder tamped with a flat or essentially flat punch or a dished (e.g., dimpled) punch. In another example, a troche has a dimpled adhesive face and a rounded ingredient face. An example of a bi-layer tablet is the adhering xylitol troche disclosed in US patent application number 11/800381, filed May 4, 2007, which is incorporated in its entirety.

The bilayer, adhering troches may be supplied in a kit with instructions for use. For use while sleeping, the troche is best not adhered to the roof of the mouth for safety reasons, because the person might pry it loose with their tongue while sleeping in which case it could fall into the airway. Instead, it is preferably adhered to the outside of a molar or adjoining gums. One or more troches, e.g., two, three, four, five or more, may be used during sleeping.

To verify that the described method of reducing reflux while sleeping works, the inventor supplied adhering troches as described above to a person who usually tastes the sourness of stomach acid in their mouth during the last 1-2 hours of sleep before waking. The person adhered two troches in their mouth each night at bedtime, one on each side of the mouth. Each troche released 500 mg of xylitol and no other flavor. During each night of sleep when the troches were used, the person reported no sour taste in his mouth during the night. When the person stopped using the troches for a night, he again reported tasting the sour stomach acid during the last hour of sleep before arising.

## Claims

1. An adhering, bilayer troche for use in a method of reducing reflux from a stomach toward a mouth while sleeping in a patient in need thereof wherein:
a. said adhering, bilayer troche is provided to the patient, the troche comprising:
i. a first layer comprising an adhesive powder, and
ii. a second layer comprising a substrate that slowly dissolves or erodes in saliva and an ingredient to be released, wherein the ingredient stimulates saliva production in the mouth; and
b. the patient is instructed to adhere one or more of the troches in their mouth before going to sleep, thereby causing frequent swallowing of stimulated saliva while sleeping resulting in reduced reflux from the stomach.

2. The troche for the use of claim 1 where the ingredient that stimulates saliva production comprises flavor molecules.

3. The troche for the use of claim 2 wherein the flavor molecules comprise a sweet polyol.

4. The troche for the use of claim 3 wherein the sweet polyol molecules are selected from the group consisting of xylitol, sorbitol, maltitol, erythritol and mannitol.

5. The troche for the use of claim 2, wherein the flavor molecules comprise a synthetic sweetener.

6. The troche for the use of claim 5, wherein the synthetic sweetener molecules are selected from the group consisting of sucralose, neotame, aspartame, acesulfame potassium and saccharin.

7. The troche for the use of claim 2, wherein the flavor molecules comprise stevia.

8. The troche for the use of claim 1, wherein the person is instructed to adhere the troche to an outside of a molar or gum adjoining the outside of the molar.

9. A slowly dissolving, adhering, bilayer troche for use in a method of reducing reflux from a stomach toward a patient's mouth while sleeping, wherein said method comprises: adhering in the mouth before going to sleep said troche that dissolves while sleeping and releases an ingredient that stimulates saliva production while sleeping, thereby causing frequent swallowing of stimulated saliva resulting in reduced reflux from the stomach, wherein the troche comprises:
i. a first layer comprising an adhesive powder, and
ii. a second layer comprising a substrate that slowly dissolves or erodes in saliva and an ingredient to be released, wherein the ingredient stimulates saliva production in the mouth.

10. The troche for the use of claim 9, wherein the ingredient comprises flavor molecules.

11. The troche for the use of claim 10, wherein the flavor molecules comprise a sweet polyol.

12. The troche for the use of claim 11, wherein the sweet polyol molecules are selected from the group consisting of xylitol, sorbitol, maltitol, erythritol and mannitol.

13. The troche for the use of claim 10, wherein the flavor molecules comprise a synthetic sweetener.

14. The troche for the use of claim 13, wherein the synthetic sweetener molecules are selected from the group consisting of sucralose, neotame, aspartame, acesulfame potassium and saccharin.

15. The troche for the use of claim 10, wherein the flavor molecules comprise stevia.

16. The troche for the use of claim 9, wherein the adhering troche is adhered to an outside of a molar or gums adjoining the outside of the molar.

## Patentansprüche

1. Anhaftende, zweischichtige Pastille zur Verwendung in einem Verfahren zum Verringern von Reflux aus einem Magen in Richtung eines Mundes während des Schlafens bei einem Patienten, der dies benötigt, wobei:
a. dem Patienten die anhaftende, zweischichtige Pastille bereitgestellt wird, wobei die Pastille umfasst:
i. eine erste Schicht, die ein Haftpulver umfasst, und
ii. eine zweite Schicht, die ein Substrat, das sich in Speichel langsam auflöst oder erodiert, und einen freizusetzenden Inhaltsstoff umfasst, wobei der Inhaltsstoff eine Speichelproduktion in dem Mund anregt; und
b. der Patient angewiesen wird, eine oder mehrere der Pastillen vor den Schlafengehen in seinem Mund anzuheften, wodurch ein häufiges Schlucken von angeregtem Speichel während des Schlafens verursacht wird, was zu einem verringerten Reflux aus dem Magen führt.

2. Pastille zur Verwendung nach Anspruch 1, wobei der Inhaltsstoff, der die Speichelproduktion anregt, Geschmacksmoleküle umfasst.

3. Pastille zur Verwendung nach Anspruch 2, wobei die Geschmacksmoleküle ein süßes Polyol umfassen.

4. Pastille zur Verwendung nach Anspruch 3, wobei die Moleküle des süßen Polyols aus der Gruppe bestehend aus Xylitol, Sorbitol, Maltitol, Erythritol und Mannitol ausgewählt sind.

5. Pastille zur Verwendung nach Anspruch 2, wobei die Geschmacksmoleküle einen synthetischen Süßstoff umfassen.

6. Pastille zur Verwendung nach Anspruch 5, wobei die Moleküle des synthetischen Süßstoffs aus der Gruppe bestehend aus Sucralose, Neotam, Aspartam, Acesulfam-Kalium und Saccharin ausgewählt sind.

7. Pastille zur Verwendung nach Anspruch 2, wobei die Geschmacksmoleküle Stevia umfassen.

8. Pastille zur Verwendung nach Anspruch 1, wobei die Person angewiesen wird, die Pastille an einer Außenseite eines Backenzahns oder an Zahnfleisch, das an die Außenseite des Backenzahns angrenzt, anzuheften.

9. Sich langsam auflösende, anhaftende, zweischichtige Pastille zur Verwendung in einem Verfahren zum Verringern von Reflux aus einem Magen in Richtung eines Mundes eines Patienten während des Schlafens, wobei das Verfahren umfasst: Anheften der Pastille vor dem Schlafengehen in dem Mund, wobei sich die Pastille während des Schlafens auflöst und einen Inhaltsstoff freisetzt, der eine Speichelproduktion während des Schlafens anregt, wodurch ein häufiges Schlucken von angeregtem Speichel verursacht wird, was zu einem verringerten Reflux aus dem Magen führt, wobei die Pastille umfasst:
i. eine erste Schicht, die ein Haftpulver umfasst, und
ii. eine zweite Schicht, die ein Substrat, das sich in Speichel langsam auflöst oder erodiert, und einen freizusetzenden Inhaltsstoff umfasst, wobei der Inhaltsstoff eine Speichelproduktion in dem Mund anregt.

10. Pastille zur Verwendung nach Anspruch 9, wobei der Inhaltsstoff Geschmacksmoleküle umfasst.

11. Pastille zur Verwendung nach Anspruch 10, wobei die Geschmacksmoleküle ein süßes Polyol umfassen.

12. Pastille zur Verwendung nach Anspruch 11, wobei die Moleküle des süßen Polyols aus der Gruppe bestehend aus Xylitol, Sorbitol, Maltitol, Erythritol und Mannitol ausgewählt sind.

13. Pastille zur Verwendung nach Anspruch 10, wobei die Geschmacksmoleküle einen synthetischen Süßstoff umfassen.

14. Pastille zur Verwendung nach Anspruch 13, wobei die Moleküle des synthetischen Süßstoffs aus der Gruppe bestehend aus Sucralose, Neotam, Aspartam, Acesulfam-Kalium und Saccharin ausgewählt sind.

15. Pastille zur Verwendung nach Anspruch 10, wobei die Geschmacksmoleküle Stevia umfassen.

16. Pastille zur Verwendung nach Anspruch 9, wobei die anhaftende Pastille an einer Außenseite eines Backenzahns oder an Zahnfleisch, das an die Außenseite des Backenzahns angrenzt, angeheftet wird.

## Revendications

1. Pastille bicouche adhérente pour son utilisation dans un procédé de réduction d'un reflux de l'estomac vers la bouche pendant le sommeil chez un patient le nécessitant, dans laquelle :
a. ladite pastille bicouche adhérente est fournie au patient, la pastille comprenant :
i. une première couche comprenant une poudre adhésive, et
ii. une seconde couche comprenant un substrat qui se dissout lentement ou s'érode dans la salive et un ingrédient à libérer, dans laquelle l'ingrédient stimule la production de salive dans la bouche ; et
b. le patient reçoit l'instruction de faire adhérer une ou plusieurs des pastilles dans sa bouche avant de se coucher, provoquant ainsi la déglutition fréquente de salive stimulée pendant le sommeil résultant en un reflux réduit de l'estomac.

2. Pastille pour son utilisation selon la revendication 1 dans laquelle l'ingrédient qui stimule la production de salive comprend des molécules aromatiques.

3. Pastille pour son utilisation selon la revendication 2 dans laquelle les molécules aromatiques comprennent un polyol à goût sucré.

4. Pastille pour son utilisation selon la revendication 3 dans laquelle les molécules de polyol à goût sucré sont sélectionnées dans le groupe consistant en le xylitol, le sorbitol, le maltitol, l'érythritol et le mannitol.

5. Pastille pour son utilisation selon la revendication 2, dans laquelle les molécules aromatiques comprennent un édulcorant synthétique.

6. Pastille pour son utilisation selon la revendication 5, dans laquelle les molécules d'édulcorant synthétique sont sélectionnées dans le groupe consistant en le sucralose, le néotame, l'aspartame, l'acésulfame, le potassium et la saccharine.

7. Pastille pour son utilisation selon la revendication 2, dans laquelle les molécules aromatiques comprennent du stévia.

8. Pastille pour son utilisation selon la revendication 1, dans laquelle l'individu reçoit l'instruction de coller la pastille sur un extérieur d'une molaire ou sur une gencive adjacente à l'extérieur de la molaire.

9. Pastille bicouche adhérente à dissolution lente pour son utilisation dans un procédé de réduction d'un reflux de l'estomac vers la bouche d'un patient pendant son sommeil, dans laquelle ledit procédé comprend : le fait coller dans la bouche avant d'aller se coucher ladite pastille qui se dissout pendant le sommeil et libère un ingrédient qui stimule la production de salive pendant le sommeil, provoquant ainsi la déglutition fréquente de salive stimulée pendant le sommeil résultant en un reflux réduit depuis l'estomac, dans laquelle la pastille comprend :
i. une première couche comprenant une poudre adhésive, et
ii. une seconde couche comprenant un substrat qui se dissout lentement ou s'érode dans la salive et un ingrédient à libérer, dans laquelle l'ingrédient stimule la production de salive dans la bouche.

10. Pastille pour son utilisation selon la revendication 9, dans laquelle l'ingrédient comprend des molécules aromatiques.

11. Pastille pour son utilisation selon la revendication 10, dans laquelle les molécules aromatiques comprennent un polyol à goût sucré.

12. Pastille pour son utilisation selon la revendication 11, dans laquelle les molécules de polyol à goût sucré sont sélectionnées dans le groupe consistant en le xylitol, le sorbitol, le maltitol, l'érythritol et le mannitol.

13. Pastille pour son utilisation selon la revendication 10, dans laquelle les molécules aromatiques comprennent un édulcorant synthétique.

14. Pastille pour son utilisation selon la revendication 13, dans laquelle les molécules d'édulcorant synthétique sont sélectionnées dans le groupe consistant en le sucralose, le néotame, l'aspartame, l'acésulfame, le potassium et la saccharine.

15. Pastille pour son utilisation selon la revendication 10, dans laquelle les molécules aromatiques comprennent du stévia.

16. Pastille pour son utilisation selon la revendication 9, dans laquelle la pastille adhérente est collée sur un extérieur d'une molaire ou sur des gencives adjacentes à l'extérieur de la molaire.
